# EUROPEAN PATENT APPLICATION

(11) **EP 1 243 645 A1**
(43) Date of publication of application: **25.09.2002**
(21) Application number: 00981387.4
(22) Date of filing: 07.12.2000
(51) Int. Cl.: C12N 1/15, C12N 15/80, C07K 14/385, C12P 37/00, C12R 1/66, C12R 1/82

(54) **PENICILLIN PRODUCTION USING TRANSGENIC MERODIPLOID STRAINS**

(30) Priority: 10.12.1999 ES 9902706
(71) Applicant: CONSEJO SUPERIOR INVESTIGACIONES CIENTIFICAS (CSIC), 28006 Madrid (ES); University of Sheffield, Sheffield, South Yorkshire S10 26J (GB); Imperial College of Science and Medecine, Londres, Greater London W12 0NN (GB)
(72) Inventor: Suarez Gonzalez, Teresa, Ctro. De Invest. Bio., 28006 Madrid (ES); Turner, Geoffrey, Sheffield, South Yorkshire S10 26J (GB); Arst, Herbert, Imperial College of Sc. and Med., London W12 0NN (GB); Penalva Soto, Miguel Angel, Ctro. De Invest.Bio., 28006 Madrid (ES)
(74) Representative: Cobas Horcajo, Susana
(86) International application number: ES0000464
(87) International publication number: WO01042426

(57) **Abstract**

The object of this invention is a procedure for obtaining a genetically altered merodiploid strain of Penicillium chrysogenum in which the activity of a regulating gene that controls penicillin biosynthesis has been altered, so that this strain is an overproducer of penicillin. This invention represents the first case in which the biosynthesis of a metabolite of industrial interest has been increased by the manipulation of a regulating gene, and therefore is a considerable novelty compared with the previously used technologies.

## Description

### SECTOR

Biotechnology. Genetic engineering of micro-organisms. Synthesis of antibiotics. Penicillin.

### STATE OF THE ART

Both *Penicillium chrysogenum* and the phylogenetically related fungus *Aspergillus nidulans* synthesise benzylpenicillin (penicillin G) from the same amino acid precursors and phenylacetate. To date, the production of penicillin has been improved by genetic engineering in these fungi by, for example:
(i) An increase in the expression of two or more of the three structural genes responsible for converting the amino acid precursors and phenylacetyl-CoA into penicillin G (Peñalva, M.A. et al. (1998) The optimisation of penicillin biosynthesis in fungi. *Trends in Biotechnology* 16: 483-489).
(ii) The interruption (in *A. Nidulans*) of the catabolic route of phenylacetate and its consequent channelling to the biosynthesis of penicillin (Mingot, J.M. et al. (1999) Disruption of *phacA*, an *Aspergillus nidulans* gene encoding a novel cytochrome P450 monooxygenase catalysing phenylacetate 2-hydroxylation, results in penicillin overproduction. *J. Biol Chem* 274: 14545-14550; also Spanish patent P97700833).
(iii) The overexpression (in *P. Chrysogenum)* of a phenylacetyl-CoA ligase of bacterial origin (Minambres, B. Et al. (1996) Molecular cloning and expression in different microbes of the DNA encoding *Psuedomonas putida* U phenylacetyl-CoA ligase - Use of this gene to improve the rate of benzylpenicillin biosynthesis in *Penicillium chrysogenum. J. Biol Chem* 271: 33531-33538; also patent P9600664), and
   (i) The channelling of the -aminoadipic acid precursor to the biosynthesis of penicillin by disruption of the biosynthesis of lysine from said precursor (Casqueiro, J. Et al. (1999) Gene targeting in *Penicillium chrysogenum:* disruption of the *lys2* gene leads to penicillin overproduction. *J Bacteriol* 181: 1181-1188).

All these strategies are based on the function gain (by overexpression) or the inactivation of structural genes involved in the biosynthesis of penicillin precursors considered individually. This invention covers an alternative possibility that had not been previously explored, which is the manipulation by genetic engineering of a regulating gene to simultaneously increase the function of several structural genes under its control. The advantage is that is can be generally applied to other secondary metabolites and does not require the prior identification of the structural genes the expression of which is altered in the receiving organism.

In *A. nidulans* (Espeso, E.A: et al. (1993) pH regulation is a major determinant in expression of a fungal penicillin biosynthetic gene. *EMBO J*. 12:3947-3956) and *P. chrysogenum* (Suárez, T and Peñalva, ,M.S. (1996) Characterisation of a *Penicillium chrysogenum* gene encoding a PacC transcription factor and its binding sites in the divergent pcbAB-pcbC promoter of t he penicillin biosynthetic cluster. *Mol. Microbiol*. 20: 529-540), the penicillin biosynthesis route is positively regulated by the zinc finger protein PacC (Tilburn, J. Et al. (1995) The *Aspergillus* PacC zinc finger transcription factor mediates regulation of both acid- and alkaline-expressed genes by ambient pH. *EMBO J.* 14: 779-790). In *A. nidulans,* PacC is synthesised in an inactive way (674 amino acidic residues) and it is activated by the proteolytic elimination of around 400 amino acids in its carboxy-terminal region. The resulting protein (approximately 250 residues) is a transcription factor of the penicillin biosynthesis genes (Tiburn, J. Et al. (1995) The *Aspergillus* PacC zinc finger transcription factor mediates regulation of both acid- and alkaline-expressed genes by ambient pH. EMBO J. 14: 779 - 790; Orejas, M. Et al. (1995) Activation of the *Aspergillus* PacC transcription factor in response to alkaline ambient pH requires proteolysis of the carboxy-terminal moiety. *Gene Develop. 9:* 1622 - 1632). This proteolytic process is produced in response to a signal that is transmitted when the environment is alkaline.

In *A. nidulans*, mutations (called PacC^{c}) in the pacC gene that produce a truncation in the carboxy-terminal region of the protein between amino acids 263 and 574 (considering the ATG codon in position 5 of the ORF as the main point of transduction initiation) cause the proteolytic activation of the protein, independent of the ambient pH, mimic alkaline ambient conditions and result in a gain in the genic pacC function, so that, whatever the ambient pH, a high expression is produced of the genes that should function with an alkaline pH and a reduced expression of the genes that should function with an acid pH. The "alkaline" genes, the expression of which is increased in this genetic fund, include penicillin biosynthesis route genes.

In *A. nidulans,* the pacC^{c} mutations behave as codominants in heterozygotic diploids with a wild-type pacC⁺ allele. The behaviour of these mutations in merodiploid strains with two copies of the pacC gene (one of them pacC^{c} and the other pacC⁺) and the rest of the genome in its normal haploid condition is unknown.

In the industrial organism *Penicillium chrysogenum* there is a homologue of the *A. nidulans* pacC gene (Suárez, T. And Peñalva, M.A. (1996) Characterisation of a *Penicillium chrysogenum* gene encoding a PacC transcription factor and its binding sites in the divergent pcbAB-pcbC promoter of the penicillin biosynthetic cluster. *Mol. Micriobiol.* 20: 529 - 540). It is possible that this gene (which we will call Pc-pacC) positively regulates the biosynthetic route of penicillin G, in which case an increase in the function could result in an increase in the levels of production of this antibiotic. Pc-pacC (GenBank ID U44726) encodes for a transcription factor (Pc-PacC) that has approximately 64% of amino acid sequence identity with its *A. nidulans* homologue. The sequence of 641 amino acids deducted for Pc-PacC is shown in SEQ. ID NO 1.

Genetic engineering technology is not very developed in the industrial organism *Penicillium chrysogenum* in relation to the model organism *A. nidulans.* It has been shown, for example, that the genic inactivation of a gene by homologous recombination with null alleles is highly inefficient (Peñalva, M.A. et al. (1998) The optimisation of penicillin biosynthesis in fungi. *Trends in biotechnology* 16: 483 - 489).

### DESCRIPTION OF THE INVENTION

### Brief description

The object of this invention is the design by genetic engineering of a genetically altered merodiploid strain of *Penicillium chrysogenum,* in which the activity of a regulating gene that controls penicillin synthesis has been altered. This invention is the first case in which penicillin biosynthesis has been improved by the manipulation of a regulating gene, and therefore is notably novel in relation to the technologies previously used.

To avoid the previously mentioned technological difficulties, this invention refers to the generation of merodiploid strains that contain, in addition to a wild-type copy of the pacC gene, one or more additional copies of a mutant version of the pacC gene that encodes for a protein truncated at amino acid 477. All these merodiploid strains are notably overproducers of penicillin in relation to the original strain and show high transcription levels of at least two genes of the biosynthesis of the antibiotic, thus demonstrating the validity of the approach followed.

### Detailed description of the invention

A series of merodiploid strains derived from *P. chrysogenum* NRRL1951. The wild-type PacC gene of this strain encodes a protein of 641 amino acids, the sequence of which is shown on SEQ ID NO 1. In addition to the wild-type pacC gene, this genetically altered strains contain one or several copies of pacC33, an allelic variant of pacC with 481 residues, which encodes for a normal PacC protein up to residue 477, after which, because of a change in the reading pattern that results from truncation, four abnormal amino acids are added, with which it ends at its carboxy-terminal end (see SEQ ID NO 3). The sequence of cDNA nucleotides that this truncated protein encodes is presented in SEQ ID NO 2). The *P. chrysogenum* pacC gene has an intron starting at nucleotide 223 of 56 pb (not included in SEQ ID NO 2; Suárez, T. and Peñalva, M.A. (1996) Characterisation of a *Penicillium chrysogenum* gene encoding a PacC transcription factor and its binding sites in the divergent pcbAB-pcbC promoter of the penicillin biosynthetic cluster. *Mol. Microbial.* 20: 529 - 540). This truncated protein is designed to provide a gain in the PacC function, through analogy with the A. *nidulans* situation, regardless of the presence or absence of the signal transduced by the pal gene route.

### Selection of a receiving P. chrysogenum strain and transformation markers

The transformation marker used to construct several of the genetically altered strains was the sC gene, which encodes for the ATP-sulphurylase enzyme, which converts the sulphate in adenosine 5'-phosphosulphate. This conversion is essential for the use of inorganic sulphate as the only source of sulphur by *P. chrysogenum* and other fungi, so that sC mutants are incapable of growing in a media with sulphate as a source of sulphur, although they normally do so in a media supplemented with sources of organic sulphate, such as L- or D-methionine. The selection of the transformants in a genetic Sc fund is based on their capacity to grow in a media with sulphate, which distinguishes them from the sC parent strain.

The selenate (SeO₄²⁻) that penetrates in the cells through the sulphate permease is a toxic compound for fungi. For example, it inhibits the growth of *P. chrysogenum* NRRL1951 at a concentration of 10 mM, isolating resistant mutants, that can have loss of function mutations in the sB gene (which encodes for the sulphate and selenate permease) or in the sC gene. These two mutant classes are distinguished, for example, because the sB mutants can grow using choline sulphate as the only source of sulphur, whereas the SC mutants do not. Therefore, in the receiving strain, spontaneously selenate-resistant mutants were selected. Once the mutant clones are isolated and purified, we analysed their capacity for growth in different compounds as a source of sulphur to diagnose the inactivated gene in each case by the mutation that created selenate-resistance (H. N. Arst, Jr. (1968) Genetic analysis of the final steps of sulphate metabolism in *Aspergillus nidulans. Nature* 219: 268 - 270).

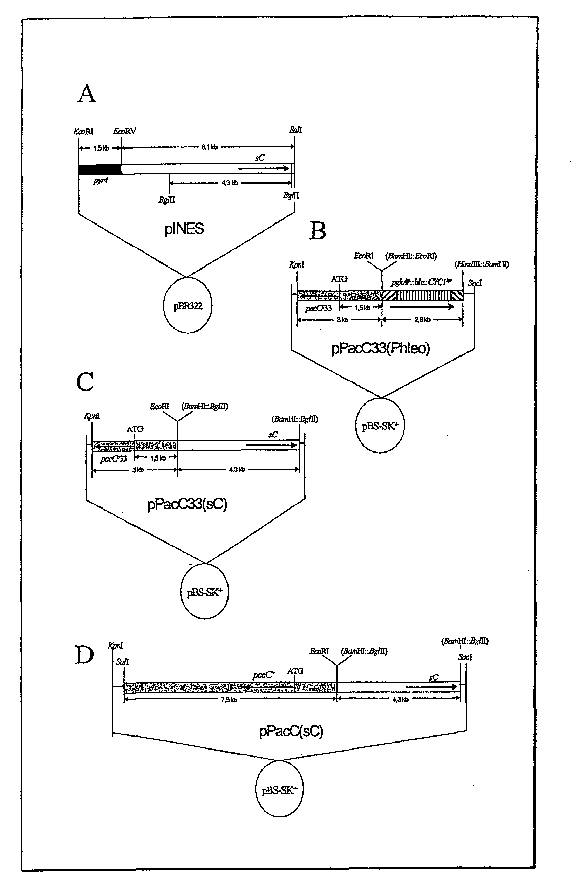

We thus identified several mutants presumably affected in the sC gene. We calculated the reversion frequency of five of these mutants and selected two of them that reverted with a frequency lower than 2x10⁻⁷. The functional sC gene of *P. chrysogenum* present in the plasmid pINES1 (Figure 1A) complemented the respective mutations present in these strains, which confirmed that they were both sC mutants. From these, we selected the one with the highest transformation frequency (45 transformations per g of pINES1). The mutant sC allele from this strain was called sC14. This strain (sC14), which was used as transformation receiver, has been deposited in the CECT with number 20327.

We also used as a transformation marker a chimeric gene in which the phleomycin-resistant bacterial ble gene (antibiotic to which *P. chrysogenum* is sensitive) is expressed under the control of transcription promoter sequences from the gpdA promoter of *A. Nidulans* and the terminator of the *Saccharomyces cerevisiae* CYC1 gene. The use of this chimeric gene, which behaves as a dominant marker for the selection of transformants in *P. Chrysogenum*, has been described by Kolar (Kolar, M. Et al. (1988) Transformation of *Penicillium chrysogenum*, using dominant selection markers and expression of an *Escherichia coli* lacZ fusion gene. *Gene* 62: 127 - 134). Prior experiments (see Examples) established that a concentration of 1 g/ml of phleomycin was optimum, for the selection of transformants in the NRRL1951 wild-type strain.

### Preparation of the transformant plasmids, transformation and selection of the genetically altered strains

A mutant Pc-pacC gene (SEQ ID NO 2) that encodes for a truncated protein with 481 amino acids (SEQ ID NO 3) was introduced in the pPhleo and pPcsC vectors to give rise to the recombinant plasmids pPacC33 (Phleo) and pPacC33 (sC), respectively (Fig. 1B and C). This mutant pc-pacC allele was called pacC33 and carries 1550 pb of the promoter region of the Pc-pacC gene upstream of the ATG transduction initiator. The Pc-pacC gene promoter is a weak promoter (Suárez, T. And Peñalva, M. A. (1996) Characterisation of a *Penicillium chrysogenum* gene encoding a PacC transcription factor and its binding sites in the divergent pcbAB-pcbC promoter of the penicillin biosynthetic cluster. *Mol. Microbiol.* 20: 529 - 540). As a control, we constructed the plasmid pPacC(sC) which is different from pPacC33(sC) in that it has a Pc-pacC⁺ allele instead of pacC33 (see Figure 1D, plasmid map).

The plasmids we introduced by transformation in *P*. *chrysogenum* NRRL1951 (pPacC33(Phleo)) or in its derived mutant strain sC14 (pPacC33(sC) and pPacC(sC)). After the selection and purification of the transformants and the analysis of the situation and number of copies of the plasmid integrated in the genome by the Southern technique, the following transformants were selected, and deposited in the Spanish Collection of Cultures (CECT) : **TX5** (CECT 20328) is a strain of *P. chrysogenum* with 3 copies of pPacC33 (Phleo) integrated in tandem in an undetermined position of the genome. **TSC4** (CECT 20329) is a strain of *P. chrysogenum* with a single copy of pPacC33(sC) integrated in the sC locus. **TSC7** (CECT 20330) is a strain of *P. chrysogenum* with a single copy of pPacC33(sC) integrated in the pacC locus. **TSC03** (CECT 20331) is a strain of *P. chrysogenum* with a single copy of pPacC(sC) integrated in the pacC locus. All this strains are morphologically indistinguishable from the wild-type strain NRRL1951.

### Results of the application of this technology

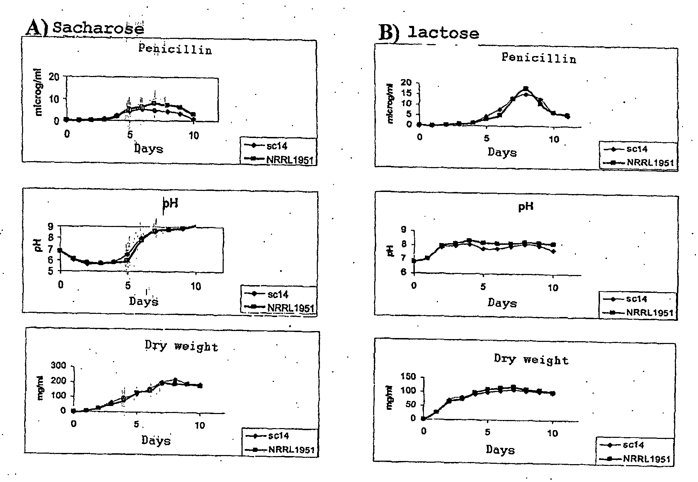

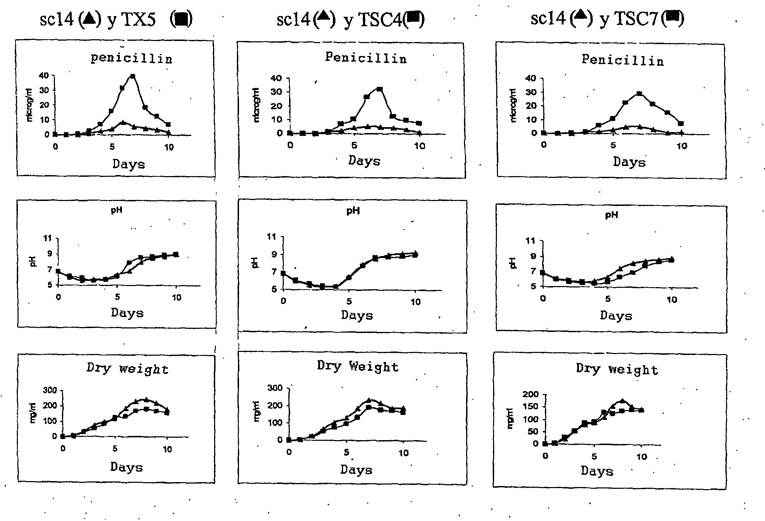

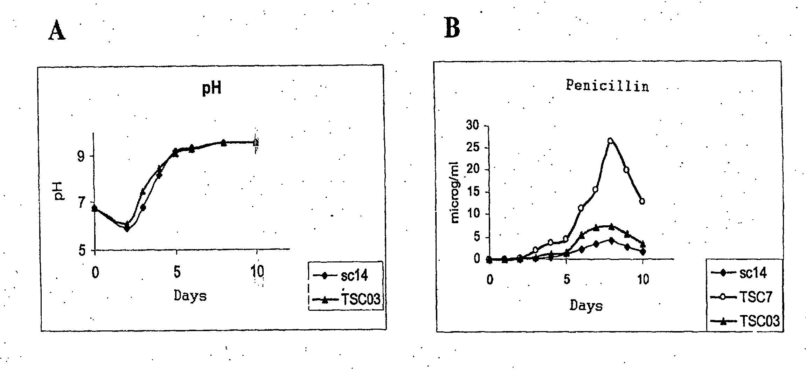

The merodiploid strains **TX5**, **TSC4** and **TSC7** are overproducers of penicillin G, in comparison with the NRRL1951 strain and with its mutant strain NRRL1951 sC14 receiver of the altered genes, with which they did not show differences in growth or in the variance of the pH of the culture media. The production of penicillin and growth rate of the sC14 strain and its parent strainNRRL1951 was very similar, showing that the sC14 mutation does not affect the production of the antibiotic (Figure 2). Using saccharose as a source of carbon (which normally inhibits production of penicillin G), the merodiploid strains reached, for example, levels at least 5 times greater than those obtained from strain sC14 (see detailed description and Figure 3). The **TSC03** strain (with two copies of the wild-type pacC gene) is also an overproducer of penicillin compared to strain sC14, although much less than, for example, strain **TSC7** with one copy of the wild-type gene and a second copy of the pacC33 allele (Figure 4). These results validate the principles used in this invention of (i) designing genetically altered strains with the pacC function increased in order to overproduce penicillin and (ii) designing pacC mutations that provide a function gain and behave as dominants in merodiploids with one copy of the wild-type pacC gene.

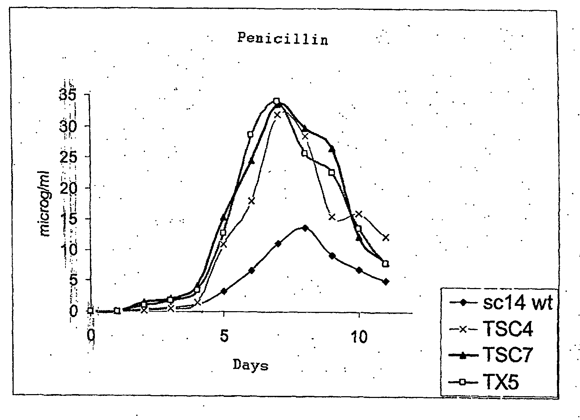

Strains **TX5**, **TSC4** and **TSC7** are also overproducers of penicillin in a lactose media, where the production levels are approximately double those obtained with sC14 or NRRL 1951 (Figure 5).

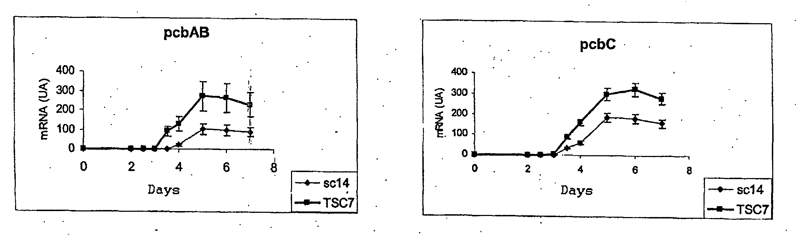

The RNA of the different transformants on different days of the penicillin G production was analysed by the Northern technique with specific probes for the structural genes pcbAB, which encodes for the ACV synthetase, and pcbC, which encodes for the IPNS synthetase, from the penicillin biosynthesis route. The quantification with Phosphorimager of the hybridation signals of the membranes, allowed us to determine the transcription profiles of these genes in the receiver strain sC14 cultivated in controlled innoculus and shaking conditions. In this strain, the transcripts of these genes were detected on day 3, with a maximum level on days 5 and 6. For example, the genetically altered strain **TSC7** increased approximately twice the times on the maximum transcription level of these two genes in comparison with the sC14 strain (Fig. 6).

To conclude, this invention describes a new procedure that increases the synthesis of penicillin by the genetic manipulation of a regulating gene, pacC. For the first time, a mutant form of this gene is presented in *P. chrysogenum*, called pacC33, which encodes a protein with a function gain and the sequence of which (SEQ ID NO 2) forms part of this invention.

This information allows us to develop other complete or partial mutant forms, both by synthesis of new DNA sequences and by the isolation and identification of natural forms, of homologue genes in other ascomycetes, which encode new proteins with a function gain in relation to the wild-type protein PacC, and form part of this invention.

The use of promoters for the expression of these mutant forms, different from the promoter of the pacC gene, be they conditional or constitutive promoters, is an evident variant of this invention and forms part of the invention.

These results show that genetically altered merodiploid strains for the pacC gene of *P. chrysogenum* that have one wild-type allele and another that is an altered pacC mutant, are strains that overproduce penicillin and have high levels of the transcripts of al least two genes involved in penicillin biosynthesis.

The regulating pacC gene controls the synthesis of other secondary metabolites and many extra-cellular enzymes. The use of this technology for the construction of genetically altered strains of *P. chrysogenum* using the strategy described here (or variants) to positively or negatively alter the pacC function in order to increase the synthesis of useful metabolites or extra-cellular enzymes or to prevent the synthesis of undesirable metabolites such as aflatoxins, is covered by this application.

The transfer of the technology described in this application to other fungi of industrial interest such as, for example, *Aspergillus niger* or *Thricoderma ressei* is evident and covered by this application.

The transfer of this technology to other ascomycete regulating genes in order to increase the synthesis of useful metabolites and extra-cellular enzymes or prevent the synthesis of undesirable metabolites is also covered by this application.

### EXAMPLES

### Example 1.- Obtaining mutant strains in the gene that encodes the ATP-sulphurylase.

The wild-type strain of *P. chrysogenum* NRRL1951 was obtained from the CBS (Holland). In order to select selenate-resistant mutants, we prepared 1.5 x 10⁸ spores of this strain on 30 minimum media dishes (Cove, D.J. (1966) The induction and repression of nitrate reductase in the fungus *Aspergillus nidulans. Biochim. Biophys*. *Act* 113: 51 - 56) with 10 mM of sodium selenate and 10 g/ml of D-methionine and 1% of glucose and 10 mM of ammonium tartrate, as sources of carbon and nitrogen, respectively. The selenate-resistant mutants appeared with a frequency of 1.5 x 10⁻⁷ spores. After purification, the phenotype of the mutants was confirmed by growth trials.

The selenate-resistant mutants can map in at least two structural genes, sC (ATP-sulphurylase) and sB (sulphate permease). The mutants in the sC gene do not grow in a media with choline sulphate as a source of S, a compound that is capable of supplementing the deficiency in the permease, since it enters the cell through a permease other than sulphate/selenate. 7 putative sC mutants were thus identified. In order to verify which of them were definitely sC mutants, we proceeded to test by transformation with the plasmid pINESI (Fig. 1A) if the mutations could be complemented by the functional sC gene of *P. chrysogenum*, by the selection of the transformants in a media with sulphate as the only source of S. After these experiments, we selected the mutant sC14, since it presented the highest transformation frequency with pINES1 (45 transformants capable of using sulphate/ g of plasmid).

### Example 2.- Determination of the sensitivity of P. Chrysogenum NRRL1951 to phleomycin.

In order to determine the sensitivity to phleomycin of the NRRL1951 strain, experiments were conducted with protoplasts in regeneration dishes, in the selection of transformant conditions. The protoplasts were obtained after incubating mycelium grown for 20 h at 25°C in a minimum media (Cove, 1966), with 30 mg of Novozyma per gram of mycelium (drained weight) for 2 h at 25°C in a 0.9 M KCl, 10 mM phosphate pH 5.8 buffer solution. The protoplasts were briefly shaken in the vortex and centrifuged at 3000g, so that the protoplasts sedimented on the undigested mycelium, from which they are distinguishable because of their whitish colour. 10⁵ feasible NRRL1951 protoplasts were extended in protoplast regeneration dishes (Cove's minimum media osmotically stabilised with 1M sorbitol and 0.M saccharose) that contained 0.05, 0.1, 0.5, 1, 5,10, 20, 40 and 50 g/ml of phleomycin (Cayla, France). At concentrations higher than 0.5 g/ml of phleomycin no colony growth was observed after 10 days of incubation at 25°. We therefore routinely used the concentration of 1 g/ml of phleomycin in the transformation experiments in which a selection based on this antibiotic has been used.

### Example 3.- Plasmids used in the transformation of NRRL1951.

The plasmid pINES1 (Figure 1A), from which the sC gene of *P. chrysogenum* was obtained, is a derivative of pBR322 that includes a 1.5 kb fragment of EcoRI-EcoRV with the pyr4 gene of *Neurospora crassa* and a 6.1 kb EcoRV-sa/1 fragment of the genomic DNA of *P. chrysogenum* that contains the sC gene. Different plasmids were constructed that carry a wild-type allele or a mutant pacC33 allele of the *P. chrysogenum* pacC gene. The presence of a Kpnl cutting site in a position of the *P. chrysogenum* gene similar to where the transduction termination triplet resulting from the pacC^{c}14 mutation in *A. nidulans* is found, allowed us to create a protein truncated at residue 477, with 4 additional residues in its carboxy-terminal before reaching a transduction termination codon (SEQ ID NO 2 and 3).

The plasmid pPacC33 (Phleo) (Figure 1B) was constructed using pBluescript II SK⁺ as a base. This vector was digested with EcoRi and Kpnl and, using conventional genetic engineering techniques, we inserted a 3037 pb EcoRI-Kpnl fragment of genomic DNA of *P. chrysogenum* NRRL1951, which includes 1553 bp of the Pc-pacC promoter and the encoding region of Pc-pacC up to codon 477 inclusive, to give rise to the pPacC33 plasmid. The resulting mutant allele (called pacC33, SEQ ID NO 2) encodes for the truncated PacC protein described in SEQ ID NO 3. In this plasmid we introduced a chimeric gene consisting on the ble gene of *E. coli* under the control of promoter signals and fungal terminators, which was obtained from the pHS103 plasmid described by Kolar (Kolar, M. et al. (1988) Transformation of *Penicillium chrysogenum* using dominant selection markers and expression of an *Escherichia coli* lacZ fusion gene. *Gene* 62: 127 - 134) as a 2.8 kb fragment of EcoRI-HindII, the cohesive ends of which were filled with the DNA polymerase of T4 to proceed to its insertion in the only BamHI site, also converted into blunt by the same method.

The plasmid pPacC33(sC) (Figure 1C) was constructed in a similar way to pPacC33 (Phleo), except that in this case we inserted in the BamHI site, instead of the phleomycin-resistant gene, an sC function gene, which was obtained from pINES as a 4.3 kb Bg/II fragment (Figure 1A). The plasmid pPacC(sC) is a derivative of pBS-SK+, in which in the first place we introduced a 7.5 kb EcoRI-Sa/I fragment, which contains the wild-type allele of the pacC gene with the same fragment of the promoter that is present in the pPacC33 (Phleo) and pPacC33(sC) plasmids. Later, the DNA fragment that contains the sC gene was introduced in the same way as in pPacC33(sC).

### Example 4.- Transformation of the NRRL1951 strain of P. chrysogenum

The transformation of *Penicillium* was conducted using the protocol described for *A. nidulans* (Tiburn, J. Et al. (1983) Transformation by integration in *Aspergillus Nidulans. Gene* 26: 205 - 211) with slight modifications. The protoplasts were obtained as described in example 2. The protoplasts were re-suspended in STC (sorbitol 1M, 10 mM Tris HCl pH 7.5, 10 mM CaCl₂), they were washed in the buffer twice and they were re-suspended at a concentration of 1-2 x 10⁷ protoplasts in 200 1 of STC. To each equal part we added 5 g of circular plasmid (in a volume of less than 20 1) and 20 1 of PEG 6000 50% (v/v) in STC, and the mixes were incubated for 20 min in ice. Then 1 ml of PEG was added to each tube and they were incubated for 5 min at 25°, 1 ml of STC was added and gently mixed, and they were centrifuged for 5 min at 12000 rpm. The protoplasts were gently re-suspended in 500 1 of STC and then sedimented by centrifugation. Finally, they were re-suspended in 200 1 of STC and extended on osmotically stabilised media dishes (Cove's minimum media (Cove, D.J. (1966) The induction and repression of nitrate reductase in the fungus *Aspergillus nidulans. Biochen. Biophys.* Act 113: 51 - 56) with 0.1 M saccharose and 1 M sorbitol), after mixing with 3 ml of the same media with a 0.25% (w/v) of agar. For the selection based on resistance to phleomycin, the antibiotic was included in the regeneration media at a concentration of 1 g/ml. For the selection based on sC, normal minimum media was used, which contains sulphate as the only source of sulphur. The dishes were incubated at 25°C. The colonies capable of growing in the selective media appeared after 6-7 days, and were purified in a non-stabilised selective media by the isolation of individual colonies that had grown from conidiospores.

### Example 5.- Molecular characterisation of the transformants

The purified transformants were grown to obtain mycelium, from which the DNA was extracted by the Pérez-Esteban method (Pérez Esteban, B. Et al. (1993) Molecular characterisation of a fungal secondary metabolism promoter: transcription of the *Aspergillus nidulans* isopenicillin N synthetase gene is modulated by upstream negative elements. *Mol. Microbiol*. 9: 881 - 895). This DNA was digested with the EcoRI or Xbal enzymes to determine the number of copies of the plasmid and its integration site in the genome. The digested DNAs were charged in 0.7% agarose gels to separate the restriction fragments, which were transferred to a nitro-cellulose membrane. This membrane was incubated for 2 h at 42°, in 50% formamide, 5X Denhart solution, 5X SSC and 0.1% SDS with 50 g/ml of sonicated salmon sperm monocatenary DNA, after which 50 ng of the corresponding probe were added: either the 2.8 kb EcoRI-HindIII fragment that contains the ble gene, or the 4.3 kb Bg/II fragment that contains the sC gene, or a 2.3 kb HindIII-KpnI fragment of *P. chrysogenum* genomic DNA belonging to the pacC gene, in all cases radioactively marked. The hybridisation was carried out for 18 h at 42°. The final washing of the filters was for 15 min at 65°C in 0.2X SSC, 0.1% SDS. The filters were exposed to self-radiographic film or Phosphorimager for radioactivity detection.

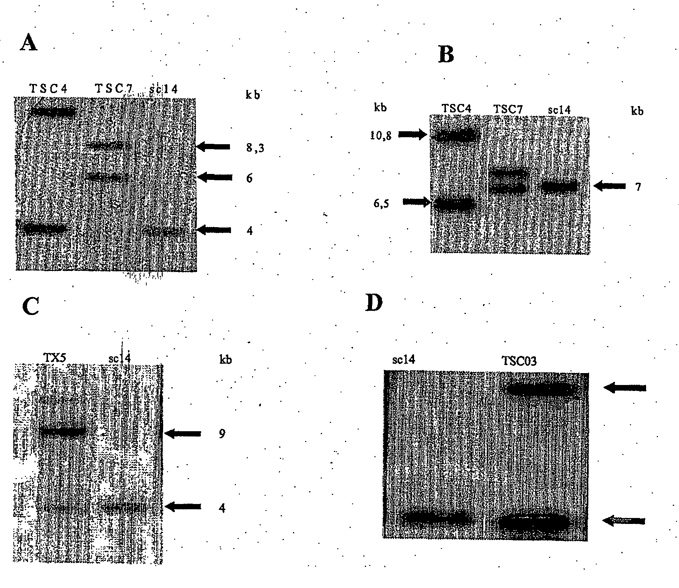

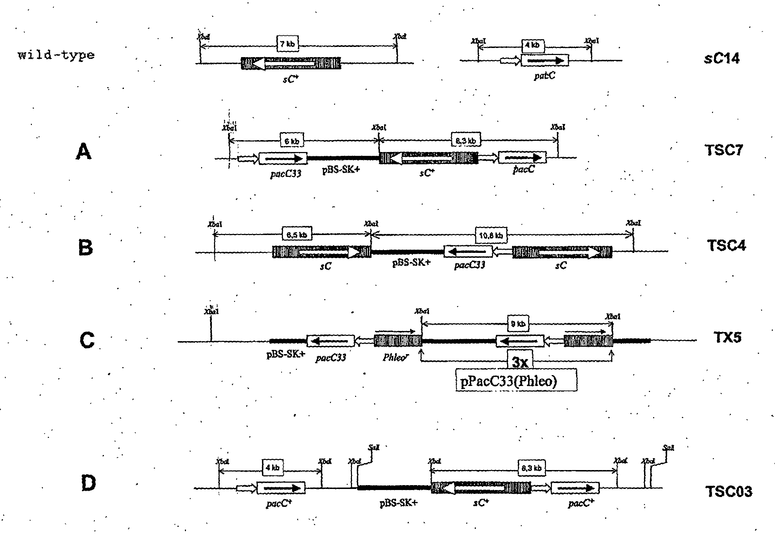

For the analysis of the transformants, digestions with Xbal were used (Figures 7 and 8). The pacC gene probe revealed a 4 kb Xbal band in the sC14 wild-type strain that is transformed into two new 6 and 8.3 kb bands in the **TSC7** transformant (in which the plasmid PacC33 (sC) is integrated in the pacC locus, Figures 7A and 8A). In the sC14 wild-type strain, the sC gene probe revealed a 7 kb band that is transformed into two 6.5 and 10.8 kb bands in the **TSC4** transformant (pPacC33(sC) integrated in locus sC, Figures 7B and 8B). The **TX5** transformant, obtained with the pPacC33 (Phleo) plasmid, presents, with the pacC gene probe, a 4 kb band, another 10-11 kb band and another close to 9 kb band (Figures 7C and 8C). This last band is three times more intense that the band from the resident pacC gene and its mobility corresponds to the size of the plasmid, so it was considered that the **TX5** merodiploid is a transformant with 3 tandem-integrated copies in an undetermined position of the genome (Figure 8C). In a similar analysis, the TSC03 transformant (Figures 7D and 8D) presents, with the pacC probe, the same 4 kb Xbal band that appears in the sC14 strain and a new 8.3 kb fragment (an internal fragment of the pacC insert of ~ 2.2 kb and the vector sequences are not detected in this hybridisation. See Figure 8D).

### Example 6.- Production of penicillin in saccharose as a source of carbon

The selected transformants, together with the control strains of NRRL 1951 or its derivative sC14, were grown at 25°C with heavy shaking in a penicillin production media (Cove's media supplemented with 2.5% (w/v) of corn steep solids and 0.12% (w/v) of sodium phenylacetate, and 3% of saccharose or 3% of lactose (both w/v) as the main source of carbon). In all cases inoculation was from a suspension of conidiospores, at an initial concentration of 1-2 x 10⁶ spores/ml, using 500 ml flasks with 100 ml of media. Media samples were taken at different times after inoculation, in which the amount of penicillin produced was measured using a biotest with *Serratia marcescens*. 1 cm diameter matrices were excavated in solid Antibiotic-1 Medium (DIFCO), which included a diluted suspension of the bacteria (at an O.D⁶⁰⁰ of 0.0075). 100 µl of an appropriate supernatant solution was introduced into these matrices. The dishes were incubated for 20 h at 37°, after which the bacterial growth inhibition halo was measured and the amount of penicillin was calculated by comparison with the halos produced by different dilutions of a standard sodium penicillin G solution.

Figure 2 shows that the level of penicillin production of the sC14 strain is very similar to that of the NRRL 1951 strain from which it is derived, indicating that the mutation does not affect production of the antibiotic. In a medium with saccharose or lactose as the main source of carbon, the growth of the different strains was very similar as far as the biomass measurement and the evolution of the extracellular pH was concerned. However, the genetically altered **TX5, TSC4** and **TSC7** strains produced significantly higher levels of penicillin than the sC14 parent strain, both with saccharose (Figure 3) and with lactose (Figure 5). In the first case, the increase in production was 4.5 times approximately, whereas in lactose, the production levels were approximately twice those obtained with sC14 or NRRL1951 (Figure 5). The **TSC03** merodiploid strain (with two copies of the pacC wild-type gene) has a growth that is similar to sC14 (see the evolution of the extracellular pH in figure 4A) and it is also an overproducer of penicillin compared to the sC14 strain (Figure 4B), although to a much lesser extent that the **TX5**, **TSC4** and **TSC7** strains, which have, in addition to a copy of the wild-type gene, one or more copies of the pacC33 allele (see Figure 4B for a comparison of the levels of penicillin production of **TSC03** with those corresponding to **TSC7** in saccharose).

### Example 7.- Transcriptional analysis of the merodiploids

Mycelium samples were taken from penicillin growth cultures over time (from day 2 to day 10). We extracted the RNA from these mycelium samples using the Lockington method (Lockington, R.A. et al. (1985) Cloning and characterisation of the ethanol utilisation regulon in *Aspergillus nidulans. Gene* 33: 137 - 149) and 10 µg of each sample were loaded on 1.2% agarose gels with 18% of formaldehyde in MOPS buffer (40 mM MOPS pH 7.2; 10 mM sodium acetate; 0.4 mM EDTA). The RNAs were transferred to nitro-cellulose membranes which were dried at 80°C to fix the RNA. These membranes were hybridised with probes that allowed us to detect the following genes: pacC (internal 1.3 kb HindIII-KpnI fragment); pcbC (internal 0.9 kb NcoL-BamHI fragment); pcbAB (2.4 kb EcoRi fragment) and the 955 bp NcoI-BamHI fragment of the acnA gene of *A. nidulans* which encodes for actin, and which was used to control the homogeneousness of the load between the different samples. The hybridisation conditions were identical to those described in example 5, except that the amount of probe added was 100 ng. The final wash was in the same buffer, but at 42°C. In all the Northern experiments a lane with 10 µg of mRNA obtained from a mycelium of the sC14 strain grown for 39 h in a penicillin production medium with 3% of lactose was included in the gel. All the nitro-cellulose membranes, after washing, were exposed for 15-18 h to a Phosphorimager screen sensitive to β emission of the P³². These screens were later read on a Phosphorimager (Molecular Dynamics) and the hybridisation signals were quantified using the ImageQuant software from the same company. In addition to hybridising the membranes with probes that reveal the problem transcripts, all the membranes were hybridised with a probe that reveals the mRNA of actin. The quantifications of the bands revealed with the pcbC, pcbAB and pacC probes were normalised with the intensity obtained on the actin band in each lane. All these quotients for a given gene of a single membrane were normalised to the common lane value (sample of the RNA of the mycelium of the sC14 strain grown in lactose for 39 h(, in order to compare the intensity measurements of different membranes. These measurements, with their standard deviation, appear in Figure 6, for the pcbAB and pcbC messenger RNAs.

### FIGURES

**Figure 1.- Restriction maps of the plasmids employed in the construction of the merodiploid strains of *P. chrysogenum*.** A) pINES plasmid; B) pPacC33 (phleo) plasmid; C) pPacC33 8sC) plasmid; and D) pPacC(sC) plasmid. The different genes are indicated as follows: black, *N. crassa* pyr4 gene; empty box, Sc gene region of *P. chrysogenum* (the arrow indicates the approximate position of the gene and the transcription direction); striped box, phleomycin-resistant gene, with the *E. coli* ble-gene in vertical stripes and the transcription promoter and terminator in slanted stripes; finally, the grey box indicates the region of the *P. chrysogenum* pacC gene, with the position of the wild-type allele and the mutant indicated with an arrow.
**Figure 2.- Growth and production of penicillin from P. *chrysogenum* NRRL1951 and sC14 strains.** The production of penicillin, pH and dry weight in NRRL 1951 and sC14 cultures in a penicillin production medium with 3% of saccharose (A) or lactose (B) as the main source of carbon.
**Figure 3.- Growth and production of penicillin from the pacC**^{**+**}**/pacC33 genetically altered strains.** The production of penicillin and the growth rate (pH and dry weight) in cultures of the genetically altered TX5, TSC4 and TSC7 strains, pacC⁺/pacC33 merodiploids, in comparison with the sC14 parent strain. The cultures were in a penicillin production medium with saccharose as the main source of carbon.
**Figure 4.- Growth and production of penicillin from the genetically altered pacC**^{**+**}**/pacC**^{**+**} **strain.** The rate of growth (extracellular pH, A) and the production of penicillin (B= from the genetically altered TSC03 strain, pacC⁺/pacC⁺ merodiploid, in comparison with the sC14 parent strain. The cultures were in a penicillin production medium with saccharose as the main source of carbon. In panel B, we also observe the difference in the production of penicillin between the pacC⁺/pacC⁺ merodiploid (TSC03) and a pacC⁺/pacC33 merodiploid (TSC7).
**Figure 5.- Production of penicillin in a media with lactose.** The production of penicillin in lactose from the pacC⁺/pacC33 merodiploid strains TSC4, TSC7 and TX5, in comparison with the sC14 parent strain.
**Figure 6.- Quantification of the pcbC and pcbAB mRNA levels in the sc14 and TSC7 strains.** The measurements are expresses in arbitrary units (AU). Culture time is indicated on abscissas. The data is the average of three experiments and the error bars indicate the standard deviation.
**Figure 7.- Southern method analysis of the merodiploid strains of *P. chrysogenum*.** The DNA samples from the different strains were digested with XbaI. The probe used was a fragment of the pacC gene (A, C and D) or the sC gene (B). The arrows indicate the hybridisation bands obtained with the merodiploids and the receiver strain, as indicated in the text.
**Figure 8.- Graphic representation of plasmid recombination.** The graphic interpretation of the bands revealed in the Southern on Figure 7 is represented here for the sc14 (wild-type), TSC7 (A), TSC4 (B), TX5 (C) and TSC03 (D) strains. The genome of the fungus is indicated by the fine continuous line. The box with thick stripes represents the sC gene (wild or mutant versions, sc14) and the white arrow indicated the transcription direction. The pacC⁺ or pacC^{c}33 mutants are represented by a white box with an internal arrow (which indicates the transcription direction). The phleomycin-resistant gene is indicated by a box with thin stripes and the plasmidic sequences by a continuous thick line. It is not possible to determine the integration site for the TX5 transformant, and we indicate the repetition of three copies of the transformant plasmid. The measurement lines show the sizes (in kb) of the fragments indicated in the hybridisation on Figure 7.

## Claims

1. Procedure for obtaining genetically altered strains of filamentous fungi **characterised in that**:
a) the genetically altered strains are merodiploids for a regulating gene,
b) it is based on the transformation of these strains with an integrating plasmid that includes a sequence of nucleotides that corresponds to a dominant or co-dominant wild-type or mutant allele of the this regulating gene, and **in that**
c) these strains present new function-gain or -loss capacities in the production of metabolites or extracellular enzymes.

2. Procedure according to claim 1, **characterised in that** there is a function gain of the regulating gene.

3. Procedure according to claim 1, **characterised in that** there is a function loss of the regulating gene.

4. Procedure according to any of claims 1 to 3, **characterised in that** the production of a secondary metabolite is increased.

5. Procedure according to any of claims 1 to 4, **characterised in that** the secondary metabolite is a penicillin or any other beta-lactamic antibiotic produced by filamentous fungi.

6. Procedure according to any of claims 1 to 5, **characterised in that** the sequence of nucleotides can be a genomic or DNAc version of the regulating gene, obtained by synthesis or selection.

7. A genetically altered merodiploid strain obtained by a procedure according to any of claims 1 to 6, where the filamentous fungus belongs to the group of ascomycetes of economic or medical interest, although it is not restricted to these, such as *Penicillium chrysogenum, Apersgillus niger, Aspergillus nidulans, Thricoderma ressei* and *Candida albicans.*

8. Sequence of nucleotides according to claim 1, **characterised in that** it represents an allele of the pacC regulating gene of *Penicillium chrysogenum* and encodes for a mutant protein with a function gain.

9. Sequence of nucleotides 'according to claim 1, **characterised in that** it represents an allele of the pacC regulating gene of *Penicillium chrysogenum* and encodes for a mutant protein with a function loss.

10. Sequence of nucleotides according to claim 8, **characterised in that** it consists of SEQ ID NO 2 and it is the pacC33 allele of the pacC regulating gene.

11. Sequence of nucleotides **characterised in that** it presents an identity of at least 30% with the sequence according to any of claims 8 to 10.

12. Plasmid according to claim 1, **characterised in that** it contains a sequence of nucleotides according to any of claims 8 to 11 and all the elements required to promote the expression of the regulating gene that corresponds to that sequence.

13. Plasmid according to claim 12, **characterised in that** the selective marker is the *P. chrysogenum* sC gene.

14. Plasmid according to claim 12, **characterised in that** the selective marker is the sC gene of others *Peniciliia, A. nidulans* and other *Aspergilli* and other filamentous or yeast-type ascomycetes.

15. Plasmid according to claim 12, **characterised in that** it presents the identifying features of the pPacC33 plasmid.

16. Plasmid according to claim 12, **characterised in that** it presents the identifying features of pPacC22(sC).

17. Plasmid according to claim 12, **characterised in that** it presents the identifying features of pPacC33(Phleo) of this invention.

18. Plasmid according to claim 12, **characterised in that** it includes a sequence of nucleotides that encodes for any function gain or loss allele of the *P. chrysogenum* pacC gene.

19. Plasmid according to claim 12, **characterised in that** it includes the sequence of nucleotides that encodes for any function gain or loss allele of the pacC regulating gene of other *Penicillia,* other *Aspergilli* and other filamentous or yeast-type ascomycetes.

20. Plasmid according to claim 1, **characterised in that** it contains the sequence of nucleotides of the wild type of the pacC regulating gene of *P. chrysogenum.*

21. Plasmid according to claim 20, **characterised by** the identifying features of the pPacC(sC) plasmid.

22. Plasmid according to claim 1, **characterised in that** it contains the sequence of nucleotides of the wild type of the pacC regulating gene of other *Penicillia,* other *Aspergilli* and other filamentous or yeast-type ascomycetes.

23. Genetically altered merodiploid strain according to claim 7, **characterised in that** it contains a plasmid that includes a wild-type or mutant allele of the pacC regulating gene.

24. Genetically altered merodiploid strain according to claim 23, **characterised in that** the mutant allele of the pacC gene is a function-gain allele.

25. Genetically altered merodiploid strain according to claim 23, **characterised in that** the mutant allele of the pacC gene is a function-loss allele.

26. Genetically altered merodiploid strain according to any of claims 7 and 23 to 25, **characterised in that** it contains a plasmid according to any of claims 12 to 22.

27. Genetically altered merodiploid strains according to claim 26, **characterised in that** they present the identifying features of the *P. chrysogenum* strains registered in the CECT with numbers 20328, 20329, 20330 and 20331.

28. Use of the genetically altered merodiploid strains according to claim 7, for the production of metabolites and extracellular proteins of industrial interest.

29. Use of the genetically altered strains according to any of claims 23 to 27 for the production of penicillin.


LIST OF SEQUENCES

<110> CONSEJO SUPERIOR DE INVESTIGACIONES CIENTIFICAS

<120> NEW REGULATING GENE OF PENICILLIN PRODUCTION

<130> PacC sequence

<140>
<141>

<160> 3

<170> PatentIn Ver. 2.1

<210> 1
<211> 641
<212> PRT
<213> Penicillium chrysogenum

<400> 1
Met Thr Glu Asn His Thr Pro Ser Thr Thr Gln Pro Thr Leu Pro Ala
1               5                   10                  15

Pro Val Ala Glu Ala Ala Pro Ile Gln Ala Asn Pro Ala Pro Ser Ala
            20                  25                  30

Ser Val Thr Ala Thr Ala Ala Thr Ala Ala Val Asn Asn Ala Pro Ser
        35                  40                  45

Met Asn Gly Ala Gly Glu Gln Leu Pro Cys Gln Trp Val Gly Cys Thr
    50                  55                  60

Glu Lys Ser Pro Thr Ala Glu Ser Leu Tyr Glu His Val Cys Glu Arg
65                  70                  75                  80

His Val Gly Arg Lys Ser Thr Asn Asn Leu Asn Leu Thr Cys Gln Trp

                85                  90                  95

Gly Thr Cys Asn Thr Thr Thr Val Lys Arg Asp His Ile Thr Ser His
            100                 105                 110

Ile Arg Val His Val Pro Leu Lys Pro His Lys Cys Asp Phe Cys Gly
        115                 120                 125

Lys Ala Phe Lys Arg Pro Gln Asp Leu Lys Lys His Val Lys Thr His
    130                 135                 140

Ala Asp Asp Ser Glu Ile Arg Ser Pro Glu Pro Gly Met Lys His Pro
145                 150                 155                 160

Asp Met Met Phe Pro Gln Asn Pro Arg Gly Ser Pro Ala Ala Thr His
                165                 170                 175

Tyr Phe Glu Ser Pro Ile Asn Gly Ile Asn Gly Gln Tyr Ser His Ala
            180                 185                 190

Pro Pro Pro Gln Tyr Tyr Gln Pro His Pro Pro Pro Gln Ala Pro Asn
        195                 200                 205

Pro His Ser Tyr Gly Asn Leu Tyr Tyr Ala Leu Ser Gln Gly Gln Glu
    210                 215                 220

Gly Gly His Pro Tyr Asp Arg Lys Arg Gly Tyr Asp Ala Leu Asn Glu
225                 230                 235                 240

Phe Phe Gly Asp Leu Lys Arg Arg Gln Phe Asp Pro Asn Ser Tyr Ala
                245                 250                 255

Ala Val Gly Gln Arg Leu Leu Gly Leu Gln Ala Leu Gln Leu Pro Phe
            260                 265                 270

Leu Ser Gly Pro Ala Pro Glu Tyr Gln Gln Met Pro Ala Pro Val Ala
        275                 280                 285

Val Gly Gly Gly Gly Gly Gly Tyr Gly Gly Gly Ala Pro Gln Pro Pro
    290                 295                 300

Gly Tyr His Leu Pro Pro Met Ser Asn Val Arg Thr Lys Asn Asp Leu
305                 310                 315                 320

Ile Asn Ile Asp Gln Phe Leu Glu Gln Met Gln Asn Thr Ile Tyr Glu
                325                 330                 335

Ser Asp Glu Asn Val Ala Ala Ala Gly Val Ala Gln Pro Gly Ala His
            340                 345                 350

Tyr Val His Gly Gly Met Asn His Arg Thr Thr His Ser Pro Pro Thr
        355                 360                 365

His Ser Arg Gln Ala Thr Leu Leu Gln Leu Pro Ser Ala Pro Met Ala
    370                 375                 380

Ala Ala Thr Ala His Ser Pro Ser Val Gly Thr Pro Ala Leu Thr Pro
385                 390                 395                 400

Pro Ser Ser Ala Gln Ser Tyr Thr Ser Asn Arg Ser Pro Ile Ser Leu
                405                 410                 415

His Ser Ser Arg Val Ser Pro Pro His Glu Glu Ala Ala Pro Gly Met
            420                 425                 430

Tyr Pro Arg Leu Pro Ala Ala Ile Cys Ala Asp Ser Met Thr Ala Gly
        435                 440                 445

Tyr Pro Thr Ala Ser Gly Ala Ala Pro Pro Ser Thr Leu Ser Gly Ala
    450                 455                 460

Tyr Asp His Asp Asp Arg Arg Arg Tyr Thr Gly Gly Thr Leu Gln Arg
465                 470                 475                 480

Ala Arg Pro Ala Glu Arg Ala Ala Thr Glu Asp Arg Met Asp Ile Ser
                485                 490                 495

Gln Asp Ser Lys His Asp Gly Glu Arg Thr Pro Lys Ala Met His Ile
            500                 505                 510

Ser Ala Ser Leu Ile Asp Pro Ala Leu Ser Gly Thr Ser Ser Asp Pro
        515                 520                 525

Glu Gln Glu Ser Ala Lys Arg Thr Ala Ala Thr Ala Thr Glu Val Ala
    530                 535                 540

Glu Arg Asp Val Asn Val Ala Trp Val Glu Lys Val Arg Leu Leu Glu
545                 550                 555                 560

Asn Leu Arg Arg Leu Val Ser Gly Leu Leu Glu Ala Gly Ser Leu Thr
                565                 570                 575

Pro Glu Tyr Gly Val Gln Thr Ser Ser Ala Ser Pro Thr Pro Gly Leu
            580                 585                 590

Asp Ala Met Glu Gly Val Glu Thr Ala Ser Val Arg Ala Ala Ser Glu
        595                 600                 605

Gln Ala Arg Glu Glu Pro Lys Ser Glu Ser Glu Gly Val Phe Tyr Pro
    610                 615                 620

Thr Leu Arg Gly Val Asp Glu Asp Glu Asp Gly Asp Ser Lys Met Pro
625                 630                 635                 640

Glu

<210> 2
<211> 1446
<212> ADN
<213> Penicillium chrysogenum

<220>
<221> CDS
<222> (1)..(1446)

<400> 2
atg acg gag aac cac acc cct tct act acg cag ccg acg ttg cct gcg    48

Met Thr Glu Asn His Thr Pro Ser Thr Thr Gln Pro Thr Leu Pro Ala
1               5                   10                  15

cct gtt gct gaa gcc gcg ccg atc caa gca aac ccg gct cct tct gcc    96

Pro Val Ala Glu Ala Ala Pro Ile Gln Ala Asn Pro Ala Pro Ser Ala
            20                  25                  30

tca gtc acg gcg act gct gct act gcg gcg gtg aac aac gcc ccc tct
144
Ser Val Thr Ala Thr Ala Ala Thr Ala Ala Val Asn Asn Ala Pro Ser
        35                  40                  45

atg aac ggc gcc ggt gag cag ttg cct tgc cag tgg gtt ggt tgc acg
192
Met Asn Gly Ala Gly Glu Gln Leu Pro Cys Gln Trp Val Gly Cys Thr
    50                  55                  60

gag aag tcc ccc act gcc gag tct cta tat gag cat gtt tgc gag cgt
240
Glu Lys Ser Pro Thr Ala Glu Ser Leu Tyr Glu His Val Cys Glu Arg
65                  70                  75                  80

cat gtt gga cgt aaa agc acc aac aac ctc aac ctg acc tgc cag tgg
288
His Val Gly Arg Lys Ser Thr Asn Asn Leu Asn Leu Thr Cys Gln Trp
                85                  90                  95

ggc act tgc aac acc aca aca gtc aag cgt gat cat atc acc tcc cac
336
Gly Thr Cys Asn Thr Thr Thr Val Lys Arg Asp His Ile Thr Ser His
            100                 105                 110

atc cgc gtt cat gtg cca ctt aag ccg cac aaa tgc gac ttt tgt ggt
384
Ile Arg Val His Val Pro Leu Lys Pro His Lys Cys Asp Phe Cys Gly
        115                 120                 125

aag gct ttc aag cgc ccc cag gat ttg aag aag cat gtc aag act cat
432
Lys Ala Phe Lys Arg Pro Gln Asp Leu Lys Lys His Val Lys Thr His
    130                 135                 140

gcg gac gac tcc gag atc cgc tcc ccc gaa ccg ggc atg aag cac cct
480
Ala Asp Asp Ser Glu Ile Arg Ser Pro Glu Pro Gly Met Lys His Pro
145                 150                 155                 160

gat atg atg ttc ccc caa aac cct agg ggt tcc cct gct gcc aca cat
528
Asp Met Met Phe Pro Gln Asn Pro Arg Gly Ser Pro Ala Ala Thr His
                165                 170                 175

tac ttc gaa agc cct atc aac ggc atc aat ggg caa tat tca cat gca
576
Tyr Phe Glu Ser Pro Ile Asn Gly Ile Asn Gly Gln Tyr Ser His Ala
            180                 185                 190

ccg cct ccc cag tac tac cag cca cac ccc cca ccc cag gct ccc aac
624
Pro Pro Pro Gln Tyr Tyr Gln Pro His Pro Pro Pro Gln Ala Pro Asn
        195                 200                 205

ccg cat tcc tac ggc aat cta tac tat gcc ctg agc caa gga caa gag
672
Pro His Ser Tyr Gly Asn Leu Tyr Tyr Ala Leu Ser Gln Gly Gln Glu
    210                 215                 220

gga ggc cac ccc tac gac cgt aag cgc gga tat gac gcg ttg aac gaa
720
Gly Gly His Pro Tyr Asp Arg Lys Arg Gly Tyr Asp Ala Leu Asn Glu
225                 230                 235                 240

ttt ttt ggc gac ttg aag cgc cgc cag ttc gac cct aat tcc tat gcc
768
Phe Phe Gly Asp Leu Lys Arg Arg Gln Phe Asp Pro Asn Ser Tyr Ala
                245                 250                 255

gcg gtc ggc cag cgt ctg ctg ggt ctc cag gcc ctt cag ctt ccc ttc
816
Ala Val Gly Gln Arg Leu Leu Gly Leu Gln Ala Leu Gln Leu Pro Phe
            260                 265                 270

ctc agt ggc cct gcc ccc gaa tac cag caa atg cct gcg cct gtt gcc
864
Leu Ser Gly Pro Ala Pro Glu Tyr Gln Gln Met Pro Ala Pro Val Ala
        275                 280                 285

gtt ggc ggc ggc ggt ggt ggt tat ggc ggt ggt gct ccc cag cct cct
912
Val Gly Gly Gly Gly Gly Gly Tyr Gly Gly Gly Ala Pro Gln Pro Pro
    290                 295                 300

ggt tac cac ctg ccc ccc atg tcc aat gtt cgg act aag aac gat ttg
960
Gly Tyr His Leu Pro Pro Met Ser Asn Val Arg Thr Lys Asn Asp Leu
305                 310                 315                 320

atc aac att gat cag ttc ctc gaa caa atg cag aac act atc tac gag
1008
Ile Asn Ile Asp Gln Phe Leu Glu Gln Met Gln Asn Thr Ile Tyr Glu
                325                 330                 335

agc gat gag aat gtg gct gct gcc ggt gtt gcc cag ccc ggc gcg cat
1056
Ser Asp Glu Asn Val Ala Ala Ala Gly Val Ala Gln Pro Gly Ala His
            340                 345                 350

tac gtg cac ggt ggc atg aat cat cgc acc acc cac tct ccc cca acc
1104
Tyr Val His Gly Gly Met Asn His Arg Thr Thr His Ser Pro Pro Thr
        355                 360                 365

cac tcc cgc caa gcc acg tta ctg caa cta cct tca gcc ccc atg gcg
1152
His Ser Arg Gln Ala Thr Leu Leu Gln Leu Pro Ser Ala Pro Met Ala
    370                 375                 380

gct gct aca gcg cac tcc cca tcg gtc ggc acc cca gcc ctg acc cca
1200
Ala Ala Thr Ala His Ser Pro Ser Val Gly Thr Pro Ala Leu Thr Pro
385                 390                 395                 400

cct tcc agc gca cag tcg tat acc tcc aac cgc tct ccc atc tcc ctg
1248
Pro Ser Ser Ala Gln Ser Tyr Thr Ser Asn Arg Ser Pro Ile Ser Leu
                405                 410                 415

cac agc tca cgc gtg tcg ccc cct cac gag gag gcg gcg ccg ggt atg
1296
His Ser Ser Arg Val Ser Pro Pro His Glu Glu Ala Ala Pro Gly Met
            420                 425                 430

tac cct cgc ttg cct gcg gcc atc tgc gcc gac agc atg act gca ggc
1344
Tyr Pro Arg Leu Pro Ala Ala Ile Cys Ala Asp Ser Met Thr Ala Gly
        435                 440                 445

tat ccg acc gcc tca ggt gcc gca cca ccc tct act ctg agc ggt gcg
1392
Tyr Pro Thr Ala Ser Gly Ala Ala Pro Pro Ser Thr Leu Ser Gly Ala
    450                 455                 460

tat gac cac gat gac cgc cgc cgc tac act ggt ggt acc caa ttc gcc
1440
Tyr Asp His Asp Asp Arg Arg Arg Tyr Thr Gly Gly Thr Gln Phe Ala
465                 470                 475                 480

cta tag
1446
Leu

<210> 3
<211> 481
<212> PRT
<213> Penicillium chrysogenum

<400> 3
Met Thr Glu Asn His Thr Pro Ser Thr Thr Gln Pro Thr Leu Pro Ala
1               5                   10                  15

Pro Val Ala Glu Ala Ala Pro Ile Gln Ala Asn Pro Ala Pro Ser Ala

            20                  25                  30

Ser Val Thr Ala Thr Ala Ala Thr Ala Ala Val Asn Asn Ala Pro Ser
        35                  40                  45

Met Asn Gly Ala Gly Glu Gln Leu Pro Cys Gln Trp Val Gly Cys Thr
    50                  55                  60

Glu Lys Ser Pro Thr Ala Glu Ser Leu Tyr Glu His Val Cys Glu Arg
65                  70                  75                  80

His Val Gly Arg Lys Ser Thr Asn Asn Leu Asn Leu Thr Cys Gln Trp
                85                  90                  95

Gly Thr Cys Asn Thr Thr Thr Val Lys Arg Asp His Ile Thr Ser His
            100                 105                 110

Ile Arg Val His Val Pro Leu Lys Pro His Lys Cys Asp Phe Cys Gly
        115                 120                 125

Lys Ala Phe Lys Arg Pro Gln Asp Leu Lys Lys His Val Lys Thr His
    130                 135                 140

Ala Asp Asp Ser Glu Ile Arg Ser Pro Glu Pro Gly Met Lys His Pro
145                 150                 155                 160

Asp Met Met Phe Pro Gln Asn Pro Arg Gly Ser Pro Ala Ala Thr His
                165                 170                 175

Tyr Phe Glu Ser Pro Ile Asn Gly Ile Asn Gly Gln Tyr Ser His Ala
            180                 185                 190

Pro Pro Pro Gln Tyr Tyr Gln Pro His Pro Pro Pro Gln Ala Pro Asn
        195                 200                 205

Pro His Ser Tyr Gly Asn Leu Tyr Tyr Ala Leu Ser Gln Gly Gln Glu
    210                 215                 220

Gly Gly His Pro Tyr Asp Arg Lys Arg Gly Tyr Asp Ala Leu Asn Glu
225                 230                 235                 240

Phe Phe Gly Asp Leu Lys Arg Arg Gln Phe Asp Pro Asn Ser Tyr Ala
                245                 250                 255

Ala Val Gly Gln Arg Leu Leu Gly Leu Gln Ala Leu Gln Leu Pro Phe
            260                 265                 270

Leu Ser Gly Pro Ala Pro Glu Tyr Gln Gln Met Pro Ala Pro Val Ala
        275                 280                 285

Val Gly Gly Gly Gly Gly Gly Tyr Gly Gly Gly Ala Pro Gln Pro Pro
    290                 295                 300

Gly Tyr His Leu Pro Pro Met Ser Asn Val Arg Thr Lys Asn Asp Leu
305                 310                 315                 320

Ile Asn Ile Asp Gln Phe Leu Glu Gln Met Gln Asn Thr Ile Tyr Glu
                325                 330                 335

Ser Asp Glu Asn Val Ala Ala Ala Gly Val Ala Gln Pro Gly Ala His
            340                 345                 350

Tyr Val His Gly Gly Met Asn His Arg Thr Thr His Ser Pro Pro Thr
        355                 360                 365

His Ser Arg Gln Ala Thr Leu Leu Gln Leu Pro Ser Ala Pro Met Ala
    370                 375                 380

Ala Ala Thr Ala His Ser Pro Ser Val Gly Thr Pro Ala Leu Thr Pro
385                 390                 395                 400

Pro Ser Ser Ala Gln Ser Tyr Thr Ser Asn Arg Ser Pro Ile Ser Leu
                405                 410                 415

His Ser Ser Arg Val Ser Pro Pro His Glu Glu Ala Ala Pro Gly Met
            420                 425                 430

Tyr Pro Arg Leu Pro Ala Ala Ile Cys Ala Asp Ser Met Thr Ala Gly
        435                 440                 445

Tyr Pro Thr Ala Ser Gly Ala Ala Pro Pro Ser Thr Leu Ser Gly Ala
    450                 455                 460

Tyr Asp His Asp Asp Arg Arg Arg Tyr Thr Gly Gly Thr Gln Phe Ala
465                 470                 475                 480

Leu